Europäisches Patentamt

(19) European Patent Office

Office européen des brevets

(11) Publication number: **0 129 634**
**B1**

# (12) EUROPEAN PATENT SPECIFICATION

(45) Date of publication of patent specification: **04.05.88**

(21) Application number: **83850180.7**

(22) Date of filing: **27.06.83**

(51) Int. Cl.⁴: **A 61 M 25/00**

(54) **An instrument for the treatment of sinusitis.**

(43) Date of publication of application:
**02.01.85 Bulletin 85/01**

(45) Publication of the grant of the patent:
**04.05.88 Bulletin 88/18**

(84) Designated Contracting States:
**DE FR GB IT NL SE**

(56) References cited:
**BE-A- 893 679**
**GB-A-2 018 600**
**US-A-3 860 006**
**US-A-3 924 633**
**US-A-4 068 659**

**Journal of Laryngology and Otology, February
1981, Vol.95 pp. 221-223, Abdel-Salan & Gibb**

(73) Proprietor: **Drettner, Börje**
**Skrovensborgs gaard**
**S-111 29 Traangsund (SE)**

(72) Inventor: **Drettner, Börje**
**Skrovensborgs gaard**
**S-111 29 Traangsund (SE)**

(74) Representative: **Grennberg, Erik Bertil et al
H ALBIHNS PATENTBYRA AB Box 7664
S-103 94 Stockholm (SE)**

**The file contains technical information
submitted after the application was filed and
not included in this specification**

Courier Press, Leamington Spa, England.

## Description

The present invention regards an instrument useful for the treatment of sinusitis, and particularly useful for repeated irrigations for treating maxillary sinusitis. More particularly, it regards an instrument for the treatment of maxillary sinusitis as recited in the preamble of claim 1.

One of the most important and efficient known methods for treating maxillary sinusitis is the drainage of purulent secretion, by means of introducing a liquid through a catheter or the like, which is introduced into the sinus, whereby the liquid and pus may exit through the existing natural lumen (ostium).

It is often necessary to renew this operation, which is unpleasant and painful particularly due to the introduction of a cannula through bone, albeit made under local anaesthesia. Therefore, instruments have been constructed where an introduced tube may be left for renewed irrigation through the same hole made in the tissue. Another reason why this is desirable is that it is advantageous to provide ventilation of the sinus when its ostium is obstructed by inflammation of the mucus.

An instrument as recited above is known from Abdel-Salam & Gibb, J. Laryngology and Otology 95 (1981) 221—223. In this known treatment, a commercially available catheter known as the Foley catheter is brought to use. A trocar and cannula are used for introducing a catheter which at its end is provided with an inflatable balloon for retention. In order to inflate the balloon, the catheter must be provided with a special lumen used only for this purpose, beside the lumen used for irrigation. Therefore, the catheter, the trocar and the cannula must have a rather large diameter, making a correspondingly large hole.

Another known instrument for treating maxillary sinusitis comprises a catheter tube provided at its end with wings which at introduction are folded together but which will fold out, roughly into a T-form after introduction by means of a trocar and a cannula (see Illum & Jeppesen, Acta Otolaryngol. 73 (1972) 506—512). Also this instrument needs a trocar of relatively large diameter, due mainly to the fact that the wings are obtained by cutting the tube, and therefore, the tube cannot be of very small diameter.

Although the known instruments can be kept in place for subsequent irrigations, (they both have anchoring means which tend to disturb venting mainly due to their size and form) it would be desirable to keep the mucous membrane as mechanically undisturbed as possible during the time when the catheter is left in place, which may be for several days. Further, it would be desirable to have a round, soft form likely not to disturb even when used in cavities of different size and form, and which is not likely to blow out when a patient sneezes.

It is therefore a first object of the invention to obtain an instrument permitting the insertion of a securable catheter by means of a piercing instrument having a diameter as small as possible. It is a second object to be able to obtain an anchorable catheter having anchoring means that are less voluminous and less prone to disturb mucous tissue than has been the case in the prior art. A third object is to obtain an inexpensive but versatile instrument.

Those objects and other objects and advantages are obtained according to the invention, by means of an instrument of the kind recited in the introduction, with the features enumerated in the characterizing clause of claim 1.

For easy manufacture, it is suitable to form the arcuate bend as a helix. The plastic tube end may then be wound a couple of turns on to a mandrel and be given a permanent tendency to keep its form by suitable heat treatment.

It is also suitable to form the end of the plastic tube as a spiral bend, e.g. in a plane perpendicular to the general direction of the tube. This will give a particularly gentle distributed anchoring likely to give minimum strain to the mucus inside the cavity.

It is preferred to utilize a plastic tube of the kind provided with é-ray absorbent matter, as this gives considerable ease in é-ray explorations.

For maximum irrigation efficiency, it is preferred to provide the arcuate portion of the plastic tube with several openings, from which irrigation liquid may exit. This also tends to improve ventilation between irrigations, as probably not all the openings will be obstructed by pus.

The invention will now be further described in relation to non-limiting embodiments thereof. Fig. 1 shows a partially sectioned cannula provided with a straightened catheter. Fig. 2 shows a plastic catheter set free, showing an example of an anchoring configuration. Fig. 3 shows another example of an anchoring configuration.

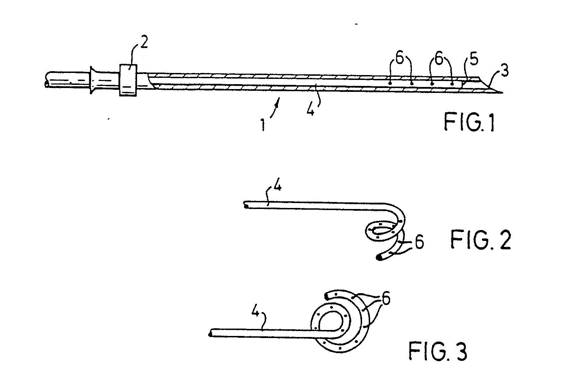

The partially sectioned view of Fig. 1 shows a cannula 1, provided with a standard Luer-Lok® fitting 2 at one end and a sharp cutting edge 3 at the other end. Inside the cannula is a plastic catheter 4 having, beside its end hole 5, several side openings 6. Although naturally not visible in Fig. 1, where the plastic catheter 4 is everywhere straight, its material is internally tensioned so that when brought out of the lumen of the cannula, the form of its end will bend into a form susceptible to its anchoring in a cavity when entered by means of the cannula. Thus, when used, the cannula is pierced through a tissue wall of a cavity, and then, the catheter 4 is moved out through the lumen and exits at 3. The cannula may be drawn back and removed, leaving the plastic catheter in place. The end of this catheter then takes an arcuate bend, exemplified in Figs. 2 and 3. Many forms are possible for the arcuate bend.

The bend as of Fig. 2 is obtained by rolling the plastic catheter over a suitable mandrel and held there during successive heating and cooling cycles, whereby the tensions created by rolling are evened out, such that the arcuate form becomes permanented.

When mounting the catheter 4 within the cannula 1, this is best performed by entering the catheter 4 from the pointed end 3. In order to facilitate this, the end of the lumen should be deburred at its inner edge, as otherwise, its edge may damage the plastic catheter 4, particularly when the arcuate form is drawn in.

In order to make the holes 6 in the catheter 4, it has been found suitable to burn them by means of a laser ray. This may be done before bending. If made before bending, the holes may be placed at will and also easily on concave sides of the arcuate portion.

According to a tested example, the cannula was of stainless steel with an outer diameter of 2,5 mm and an inner diameter of 2,1 mm, together with a plastic catheter having an outer diameter of 1,8 mm and an inner diameter of 1,5 mm. The overall length of the cannula was 11 cm.

The plastic catheter was made by taking a length of polyethylene ("Tygon"), the middle of which was wound about six turns around a mandrel having a circular form and a diameter of about 6 mm. The mandrel was dipped alternatively in near-boiling water (98°C) and in ice water three times in succession, whereby the deformation was made permanent. The helix portion was then cut off at the middle, creating two catheters. The holes had been made in the tube beforehand.

It is preferred for hygienic reasons to condition the instrument of the invention as a disposable unit. In order to facilitate its practical use, it may be packed together with plastic sacks filled with a suitable irrigation liquid and provided with suitable means for fitting to the protruding end of the anchored plastic catheter.

After insertion, the anchored plastic catheter may, after a first irrigation, be cut off a short length outside the nostril, such that it may be pushed into the nostril and out of view until next irrigation. The patient may thus be under policlinical treatment, often making hospitalization unnecessary.

## Claims

1. Equipment for the treatment of maxillary sinusitis, comprising a straight cannula (1) and a catheter (4) for entering one end thereof, by means of said cannula (1), through a nostril and a pierced hole into a maxillary sinus, said one end of said catheter (4) being provided with anchoring means (Fig. 2, 3) forming, after entering through said hole and withdrawal of said cannula (1), a shape adapted to retain said one end of the catheter in said sinus, characterized in that said cannula (1) has formed at one end a sharp cutting edge (3) for piercing said hole, is provided with an inner lumen, and in that said catheter is a plastic tube (4) insertable into said lumen and provided at its said one end with an arcuate bend (Fig. 2, 3) for constituting said anchoring means when liberated inside said sinus, said arcuate bend (Fig. 2, 3) being elastically straightened out when residing in said lumen of said cannula.

2. The equipment of Claim 1, further characterized in that said arcuate bend is a helical bend.

3. The equipment of Claim 1, further characterized in that said arcuate bend is a spiral bend.

4. The equipment of Claim 1, further characterized in that the plastic tube is made of a material comprising é-ray absorbent matter.

5. The equipment of Claim 1, further characterized in that said one end of the plastic tube is provided with several side openings (6).

## Patentansprüche

1. Ausrüstung für die Behandlung einer Oberkieferhöhlenentzündung, mit einer geraden Kanüle und einem Katheter (4) zum Einführen eines Endes des Katheters mit Hilfe der Kanüle (1) durch ein Nasenloch und ein durchgestochenes Loch in eine Oberkieferhöhle, wobei dieses eine Ende des Katheters (4) mit Verankerungsmitteln (Fig. 2, 3) versehen ist, die nach dem Einsetzen durch das Loch und Zurückziehen der Kanüle (1) eine Form bilden, die derart ausgebildet ist, daß sie dieses eine Ende des Katheters in der Höhle hält, dadurch gekennzeichnet daß die Kanüle (1) an ihrem einen Ende eine scharfe Schneidkante (3) zum Stoßen des Loches gebildet hat, mit einem inneren Lumen versehen ist, und daß der Katheter ein Plastikrohr (4) ist, das in das Lumen einsetzbar ist und an diesem einen Ende mit einer bogenförmigen Biegung (Fig. 2, 3) zur Bildung der Verankerungsmittel versehen ist, wenn dieses innerhalb der Höhle freigegeben wird, wobei die bogenförmige Biegung (Fig. 2, 3) elastisch gerade gerichtet wird, wenn sie in dem Lumen der Kanüle angeordnet ist.

2. Ausrüstung nach Anspruch 1, weiterhin dadurch gekennzeichnet, daß die bogenförmige Biegung eine schraubenförmige Krümmung ist.

3. Ausrüstung nach Anspruch 1, weiterhin dadurch gekennzeichnet, daß die bogenförmige Biegung eine Spiralkrümmung ist.

4. Ausrüstung nach Anspruch 1, weiterhin dadurch gekennzeichnet, daß das Kunststoffrohr aus einem Material hergestellt ist, das röngenstrahlabsorbierendes Material aufweist.

5. Ausrüstung nach Anspruch 1, weiterhin dadurch gekennzeichnet, daß das eine Ende des Kunststoffrohrs mit mehreren Seitenöffnungen (6) versehen ist.

## Revendications

1. Dispositif pour le traitement de la sinusite maxillaire, comportant une canule rectiligne (1) et un cathéter (4) devant pénétrer par une extrémité, au moyen de ladite canule (1), dans une narine et un trou percé dans un sinus maxillaire, ladite extrémité dudit cathéter (4) étant munie de moyens d'ancrage (Fig. 2, 3) qui prennent, après la pénétration dans ledit trou et l'enlèvement de ladite canule (1), une forme appropriée pour maintenir ladite extrémité du cathéter dans ledit sinus, caractérisé en ce que ladite canule (1) présente à une extrémité une arête de coupe vive (3) pour percer ledit trou, en ce qu'elle munie d'un passage intérieur, et en ce que ledit cathéter est un tube plastique (4) pouvant être introduit dans ledit passage et muni à sa dite extrémité d'une partie incurvée (Fig. 2, 3) pour constituer lesdits moyens d'ancrage quand il est libéré à l'intérieur dudit sinus, ladite partie incurvée (Fig. 2, 3) étant redressée élastiquement quand elle est logée dans ledit passage de ladite canule.

2. Dispositif selon la revendication 1, caractérisé en outre en ce que ladite partie incurvée est une partie hélicoïdale.

3. Dispositif selon la revendication 1, caractérisé en outre en ce que ladite partie incurvée est une partie en spirale.

4. Dispositif selon la revendication 1, caractérisé en outre en ce que le tube plastique est constitué en un matériau contenant une matière qui absorbe les rayons é.

5. Dispositif selon la revendication 1, caractérisé en outre en ce que ladite extrémité du tube plastique est munie de plusieurs orifices latéraux (6).

FIG.1

FIG.2

FIG.3